# EUROPEAN PATENT APPLICATION

(11) **EP 2 965 760 A1**
(43) Date of publication of application: **13.01.2016**
(21) Application number: 14306121.6
(22) Date of filing: 09.07.2014
(51) Int. Cl.: A61K 31/505, A61K 31/513, A61K 31/52, A61K 31/536, A61K 31/675, A61P 17/06

(54) **Combination of antiretroviral agents for use in the prevention or treatment of chronic inflammatory diseases**

(71) Applicant: Université de Montpellier, 34090 Montpellier (FR); INSERM (Institut National de la Santé et de la Recherche Scientifique), 75654 Paris Cedex 13 (FR)
(72) Inventor: Moles, Jean-Pierre, 34660 COURNONSEC (FR); Guilhou, Jean-Jacques, 34570 MURVIEL LES MONTPELLIER (FR); Dujols, Pierre, 34000 MONTPELLIER (FR); Nagot, Nicolas, 34730 PRADES LES LEZ (FR); Van De Perre, Philippe, 34730 SAINT VINCENT DE BARBEYRARGUES (FR)
(74) Representative: Monni, Richard

(57) **Abstract**

The present invention relates to the use of a composition comprising tenofovir in association with an antiviral compound for the treatment or the prevention of pathologies linked to an auto-inflammation.

## Description

The present invention relates to the use of antiretroviral agents for treating pathologies.

Reverse transcription (RT) is the key step for the spreading of infectious retroviruses. Aside of this virological function, additional RT activity are found associated with cellular proteins with either a defined function as well-illustrated by the telomerase activity or a function that remains to be explained as for the retrotransposition of endogenous retroelements.

Retroelements encompass about 40% of our genome and are divided into LTR and non-LTR elements. The former ones are present in extremely high copy numbers and are identified as short interspersed nuclear elements (SINE) and long interspersed nuclear elements (LINE). If SINE is a non-autonomous element with no protein coding capacity, LINE is of interest since the ORF2 encodes for a RT-like proteins. The LTR elements include the endogenous retroviruses (HERV, 8% of our genome) that are again subdivided into subfamilies named by their initiating t-RNA. Most of them are largely defective since they accumulated point mutations, insertions/deletions. Nevertheless, taken individually each locus possess open reading frames (ORFs) that can potentially encode for complete gag, pol or env proteins, and regulatory regions (LTRs) that kept their functional activities. Therefore, out of all known endogenous retrovirus family, the HERV-K family which integrated our genoma the most recently, is the most likely to encode for complete ORFs, and HERV-K113 and -K115 were described as the first examples of retrovirus that were endogenized and contains fully complete ORFs. Even if they got all the characters to be infectious, many others loci got individual ORF that could generate functional RT proteins that would not be involved in a classical viral cycle, but rather in an undefined cellular function or as an innocent bystander.

When analysed i.e. when not filtered through repbase listing, the expression of these RT-encoding repetitive elements is ubiquitous but differs substantially among species and among strains. Strong variations are also reported between pathological versus physiological organs and the strongest levels of expression were found in hyperproliferative pathological states such as in human cancer. For example, HERV-K expression was significantly higher in breast cancer tissues and breast cancer cell lines than in normal breast tissues and RT encoding gene were shown to be active in these cells. In addition, it turns out that RT negative tumours were of better prognostic than the RT positive tumours. Expression has also been reported in normal tissues including again variations. Seifarth et al. demonstrated that skin, thyroid gland, placenta, and tissues of reproductive organs have significantly higher levels of expression of HERV than muscle cells for example. It is also noteworthy that embryos contain also high level of RT encoding transcripts. Various reports now described the HERVs has part of a stem cell signature and their expression is tightly controlled through nucleic acid sensor, namely Toll-like receptors. RT protein and RT activity has not been, however, investigated deeply in the previous situations. This fact results from the too rare tools available for such analysis. An RT from the HERV-K family with a low activity was detected in human bone marrow cells or in spermatozoids. As an overall line, RT encoding genes are transcriptionally active in undifferentiated cells and cells with high proliferative potential while being barely detectable in differentiated cells.

However, the need of an efficient therapy for treating inflammatory diseases remains.

Initially considered as a hyperproliferative disease, antimitotic compounds were used to treat psoriasis, such as methotrexate or tar. Further, knowledges about the differentiation dysfunctions have motivated the use of pro-differentiating agents such as vitamin A or D. Moreover, highlighting of the inflammatory mechanisms has motivated the use of compounds such as cyclosporine and therapy against cytokines.

However, these treatments show numerous side effects and remain not long-term completely satisfying since some therapeutic escape are observed.

So, one aim of the invention is to provide an efficient treatment for inflammatory diseases.

For this purpose, the invention relates to a composition comprising:
- an effective amount of a first antiretroviral agent, said first antiretroviral agent being tenofovir, or a salt or a solvate, or a prodrug thereof, and
- an effective amount of at least a second antiretroviral agent, said second antiretroviral agent been chosen among the group consisting of: lamivudine, rilpivirine, efavirenz, raltegravir, or a combination thereof, or a salt or a solvate or a prodrug of the above
for its use for the prevention or the treatment of chronic inflammatory diseases.

The invention also relates to the use of a composition comprising:
- an effective amount of a first antiretroviral agent, said first antiretroviral agent being rilpivirine, or a salt or a solvate or a prodrug thereof, and
- an effective amount of at least a second antiretroviral agent, said second antiretroviral agent been chosen among the group consisting of: lamivudine, tenofovir, efavirenz, raltegravir, or a combination thereof, or a salt or a solvate or a prodrug of the above.

The invention is based on the unexpected observation made by the inventors that only specific combinations of RT inhibitors are able to efficiently and rapidly treat chronic inflammatory diseases. Indeed, the inventors have made the unexpected observation that an endogenous RT activity is increased chronic inflammatory diseases, in particular in psoriatic lesions, and consequently, inhibiting such an activity could reverse said pathologies.

The invention also relates to a method for preventing or treating chronic inflammatory diseases by administering to a patient in a need thereof a composition comprising :
- an effective amount of a first antiretroviral agent, said first antiretroviral agent being tenofovir, or a salt or a solvate, or a prodrug thereof, and
- an effective amount of at least a second antiretroviral agent, said second antiretroviral agent been chosen among the group consisting of: lamivudine, rilpivirine, efavirenz, raltegravir, or a combination thereof, or a salt or a solvate or a prodrug of the above,
or a composition comprising :
- an effective amount of a first antiretroviral agent, said first antiretroviral agent being rilpivirine, or a salt or a solvate or a prodrug thereof, and
- an effective amount of at least a second antiretroviral agent, said second antiretroviral agent been chosen among the group consisting of: lamivudine, tenofovir, efavirenz, raltegravir, or a combination thereof, or a salt or a solvate or a prodrug of the above.

The inventors have demonstrated that only specific association of nucleotidic RT inhibitors (NtNRTI) with specific nucleosidic RT inhibitors (NRTI), non-nucleosidic RT inhibitors (nNRTI), integrase inhibitors are powerful and efficient combination for treating chronic inflammatory diseases.

The composition for its use, or the method, according to the invention comprises: an effective amount of tenofovir, in association with an effective amount of at least one of the following RT inhibitors: lamivudine, rilpivirine, efavirenz, raltegravir.

The composition therefore encompasses the following combinations:
- tenofovir + lamivudine,
- tenofovir + rilpivirine,
- tenofovir + efavirenz,
- tenofovir + raltegravir,
- tenofovir + lamivudine + rilpivirine,
- tenofovir + lamivudine + efavirenz,
- tenofovir + lamivudine + raltegravir,
- tenofovir + rilpivirine + efavirenz,
- tenofovir + rilpivirine + raltegravir,
- tenofovir + efavirenz + raltegravir,
- tenofovir + lamivudine + rilpivirine + efavirenz,
- tenofovir + lamivudine + rilpivirine + raltegravir,
- tenofovir + lamivudine + efavirenz + raltegravir,
- tenofovir + rilpivirine + efavirenz + raltegravir, and
- tenofovir + lamivudine + rilpivirine + efavirenz + raltegravir.

The invention also encompasses the following combinations :
- rilpivirine + lamivudine,
- rilpivirine +tenofovir,
- rilpivirine + efavirenz,
- rilpivirine + raltegravir,
- tenofovir + lamivudine + rilpivirine,
- rilpivirine + lamivudine + efavirenz,
- rilpivirine + lamivudine + raltegravir,
- tenofovir + rilpivirine + efavirenz,
- tenofovir + rilpivirine + raltegravir,
- rilpivirine + efavirenz + raltegravir,
- tenofovir + lamivudine + rilpivirine + efavirenz,
- tenofovir + lamivudine + rilpivirine + raltegravir,
- rilpivirine + lamivudine + efavirenz + raltegravir,
- tenofovir + rilpivirine + efavirenz + raltegravir, and
- tenofovir + lamivudine + rilpivirine + efavirenz + raltegravir.

Tenofovir disoproxil fumarate is a prodrug form of tenofovir, a nucleotidic RT inhibitor. It is also marketed under the brand name Viread by Gilead Sciences. Tenofovir is also available in a fixed-dose combination with emtricitabine (the prodrug of lamivudine) in a product with the brand name Truvada for once-a-day dosing. Atripla, a fixed-dose triple combination of tenofovir, emtricitabine and efavirenz, was approved by the FDA on 12 July 2006 and is now available, providing a single daily dose for the treatment of HIV.

Rilpivirine (TMC278, trade name Edurant) is a pharmaceutical drug, developed by Tibotec and now marketed by Johnson & Johnson, for the treatment of HIV infection. It is a second-generation non-nucleoside reverse transcriptase inhibitor (NNRTI) with higher potency, longer half-life and reduced side-effect profile compared with older NNRTIs, such as efavirenz.

Efavirenz (EFV, brand names Sustiva, Stocrin, Efavir etc.) is a non-nucleoside reverse transcriptase inhibitor (NNRTI) discovered at Merck Research Laboratories. It is used as part of highly active antiretroviral therapy (HAART) for the treatment of a human immunodeficiency virus (HIV) type 1.

Raltegravir (RAL, Isentress, formerly MK-0518) is an antiretroviral drug produced by Merck & Co., used to treat HIV infection. It received approval by the U.S. Food and Drug Administration (FDA) on 12 October 2007, the first of a new class of HIV drugs, the integrase inhibitors, to receive such approval.

Emtricitabine is an analogue of cytidine. The drug works by inhibiting reverse transcriptase, the enzyme that copies HIV RNA into new viral DNA. By interfering with this process, which is central to the replication of HIV, emtricitabine can help to lower the amount of HIV, or "viral load". Emtricitabine is the prodrug of Lamivudine (2',3'-dideoxy-3'-thiacytidine, commonly called 3TC), a potent nucleosidic analog reverse transcriptase inhibitor (NRTI).

RT encoding genes have been detected in skin, as mentioned above, and increased expression were found in hyperproliferative but benign skin lesion of patient affected by psoriasis. Interestingly, psoriatic epidermis is also characterised by an abnormal differentiation program, cells were not capable to generate a mature stratum corneum. Moreover, we showed that these RT were functional and a baseline activity was detectable in normal skin extracts. The previous increase expression was followed by an increase RT activity. These observations prompted the inventors to investigate the cellular functions of RT proteins by testing various classes of RT inhibitors on different primary cell culture conditions and keratinocyte cell lines.

In the invention, it is meant that a prodrug is a medication that is administered in an inactive or less than fully active form, and then it becomes converted to its active form through a normal metabolic process, such as hydrolysis of an ester form of the drug.

Instead of administering a drug, a prodrug might be used instead to improve how a medicine is absorbed, distributed, metabolized, and excreted (ADME). Prodrugs are often designed to improve bioavailability when a drug itself is poorly absorbed from the gastrointestinal tract. A prodrug may be used to improve how selectively the drug interacts with cells or processes that are not its intended target. This reduces adverse or unintended effects of a drug, in particular in order to overcome severe unintended and unexpected side effects.

For instance in the invention, Emtracitabine is a prodrug of the lamivudine.

Advantageously, the invention relates to a composition for its use as defined above, or a method as defined above, wherein said chronic-inflammatory diseases is chosen among the group consisting of: psoriasis, sclerodermia, schizophrenia, autism, Alzheimer's disease, central nervous system inflammation, Parkinson's Disease, dementia (various forms), multiple sclerosis, Sjrögren syndrome, obesity, Hashimoto's disease, diabetes (Type 1 and Type 2), atherosclerosis, neonatal onset multisystem inflammatory disease, (also known as NOMID), Chronic Neurologic Cutaneous and Articular Syndrome (or CINCA), rheumatoid arthritis, juvenile idiopathic arthritis, osteoarthritis, systemic lupus erythematosus, cutaneous lupus erythematosus, organ inflammation (e.g. myocarditis, asthma, nephritis, colitis), inflammatory bowel disease (IBD), inflammatory bowel syndrome (IBS), Crohn's disease, primary biliary cirrhosis, Chronic Ulcerative Colitis, celiac disease, inflammatory lung disease, chronic obstructive pulmonary disease, idiopathic emphysema and idiopathic pulmonary fibrosis, Aicardi-Goutières syndrome, idiopathic autoimmune chronic active hepatitis (IACAH).

Advantageously, the invention relates to a composition for its use as defined above, or a method as defined above, wherein said auto-inflammatory disease is psoriasis.

In one advantageous embodiment, the invention relates to a composition for its use as defined above, or a method as defined above, wherein said first antiretroviral agent and said second medical agent are combined for a simultaneous, separate or sequential use.

In the context of the invention, the composition mentioned above is used for treating the above mentioned pathologies, by providing tenofovir or rilpivirine
- either by providing tenofovir or rilpivirine simultaneously with a second antiretroviral agent, said second antiretroviral agent, been chosen among the group consisting of: lamivudine, rilpivirine, efavirenz, integrase inhibitors, or a combination thereof, or a salt or a solvate or a prodrug of the above,
- or by providing tenofovir or rilpivirine and in the same time, but in another route, a second antiretroviral agent, said second antiretroviral agent, been chosen among the group consisting of: lamivudine, rilpivirine, efavirenz, integrase inhibitors, or a combination thereof, or a salt or a solvate or a prodrug of the above,
- or by providing tenofovir or rilpivirine at a determined time, and before or after, but not at the same time, a second antiretroviral agent, said second antiretroviral agent, been chosen among the group consisting of: lamivudine, rilpivirine, efavirenz, integrase inhibitors, or a combination thereof, or a salt or a solvate or a prodrug of the above.

In one another advantageous embodiment, the invention relates to a composition for its use according to the above definition, comprising :
- an effective amount of a first antiretroviral agent, said first antiretroviral agent being tenofovir or rilpivirine, or a salt or a solvate or a prodrug thereof,
- an effective amount of a second antiretroviral agent, said second antiretroviral agent, been chosen among the group consisting of: lamivudine, rilpivirine, efavirenz, integrase inhibitors, or a combination thereof, or a salt or a solvate or a prodrug of the above, and possibly :
- an effective amount of a third antiretroviral agent, said third antiretroviral agent been chosen among the group consisting of: lamivudine, rilpivirine, efavirenz, integrase inhibitors, or a combination thereof, or a salt or a solvate or a prodrug of the above,
said second and third antiretroviral agent being different.

As mentioned above, the composition for its use according to the invention encompasses advantageously the following combinations:
- tenofovir + lamivudine + rilpivirine,
- tenofovir + lamivudine + efavirenz,
- tenofovir + lamivudine + raltegravir,
- tenofovir + rilpivirine + efavirenz,
- tenofovir + rilpivirine + raltegravir,
- tenofovir + efavirenz + raltegravir,
- rilpivirine + lamivudine + efavirenz,
- rilpivirine + lamivudine + raltegravir, and
- rilpivirine + efavirenz + raltegravir.

In one another advantageous embodiment, the invention relates to a composition for its use as defined above, comprising:
- an effective amount of a first antiretroviral agent, said first antiretroviral agent being tenofovir, or a salt or a solvate or a prodrug thereof,
- an effective amount of a second antiretroviral agent, said second antiretroviral agent being lamivudine, or a salt or a solvate or a prodrug thereof, and possibly
- an effective amount of a third antiretroviral agent, said third antiretroviral agent being rilpivirine, or a salt or a solvate or a prodrug thereof.

Advantageously, the invention relates to the method as defined above, wherein the composition comprises:
- an effective amount of a first antiretroviral agent, said first antiretroviral agent being tenofovir, or a salt or a solvate or a prodrug thereof,
- an effective amount of a second antiretroviral agent, said second antiretroviral agent being lamivudine, or a salt or a solvate or a prodrug thereof, and possibly
- an effective amount of a third antiretroviral agent, said third antiretroviral agent being rilpivirine, or a salt or a solvate or a prodrug thereof.

The above combination is known as Eviplera® when the above mentioned first second and third antiretroviral agent are provided in a specific concentration or dosage.

Advantageously, the invention relates to a composition for its use according to the above definition, or the composition as defined above, wherein the effective amount of the first antiretroviral agent is from 1 µM to 10 mM.

When the first antiretroviral agent is tenofovir, advantageous amounts are from 1 µM to 1mM, advantageously from 10 µM to 100 µM. For the prevention of the above pathologies, in particular psoriasis, the most advantageous amount is 10 µM. By contrast, for a curative application, the advantageous amount is about 100 µM.

When the first antiretroviral agent is rilpivirine, advantageous amounts are from 1 µM to 250 µM, advantageously from 10 µM to 100 µM. For the prevention of the above pathologies, in particular psoriasis, the most advantageous amount is 10 µM. By contrast, for a curative application, the advantageous amount is about 100 µM.

Advantageously, the invention also relates to a composition for its use as defined above, or the method as defined above, wherein the effective amount of the second antiretroviral agent, and possibly the third antiretroviral agent, is from 1 µM to 100 µM.

In still another advantageous embodiment, the invention relates to a composition for its use as defined above, comprising
- an effective amount of a first antiretroviral agent, said first antiretroviral agent being tenofovir, or a salt or a solvate or a prodrug thereof,
- an effective amount of a second antiretroviral agent, said second antiretroviral agent being lamivudine, or a salt or a solvate or a prodrug thereof, and
- an effective amount of a third antiretroviral agent, said third antiretroviral agent being rilpivirine, or a salt or a solvate or a prodrug thereof,
wherein said chronic-inflammatory disease is psoriasis.

The invention also relates to the method as defined above for the prevention or the treatment of psoriasis, comprising the administration of an effective mount of a composition comprising :
- an effective amount of a first antiretroviral agent, said first antiretroviral agent being tenofovir, or a salt or a solvate or a prodrug thereof,
- an effective amount of a second antiretroviral agent, said second antiretroviral agent being lamivudine, or a salt or a solvate or a prodrug thereof, and
- an effective amount of a third antiretroviral agent, said third antiretroviral agent being rilpivirine, or a salt or a solvate or a prodrug thereof,

Another advantageous embodiment of the invention relates to a composition for its use according to the above definition, or the composition within the context of the method as defined above, in association with a vitamin D derivative, or a retinoid compound, or corticoid, tar and anthracene derivative, methotrexate, ciclosporin, fumaric acid derivatives, immunosuppressive treatment (such as tacrolimus or sirolimus) and any anti-cytokine/ cytokine receptor antibody (TNF-α, IL-12, IL-17, IL-12R, IL-23), wherein said pathology is psoriasis.

It is also possible to combine the above mentioned association with a PUVA therapy (a psoralen + UVA treatment) or an UVB therapy.

The invention will be better understood from the following examples and figures.

### BRIEF DESCRIPTION OF THE DRAWINGS

**Figures 1A-C** represent the expression of cytokeratin 10 on SCC12B2-derived 3D reconstructed skin detected by immunohistochemistry.
Figure 1A represents untreated SCC12B2-derived 3D reconstructed skin.
Figure 1B represents SCC12B2-derived 3D reconstructed skin treated by AZT (100 µM) for 21 days:
Figure 1C: represents SCC12B2-derived 3D reconstructed skin treated by efavirenz (100 µM) for 21 days.
   Arrow indicates cytokeratin 10 expression.
**Figures 2A-D** represent the expression of psoriasine on SCC12B2-derived 3D reconstructed skin detected by immunofluorescence.

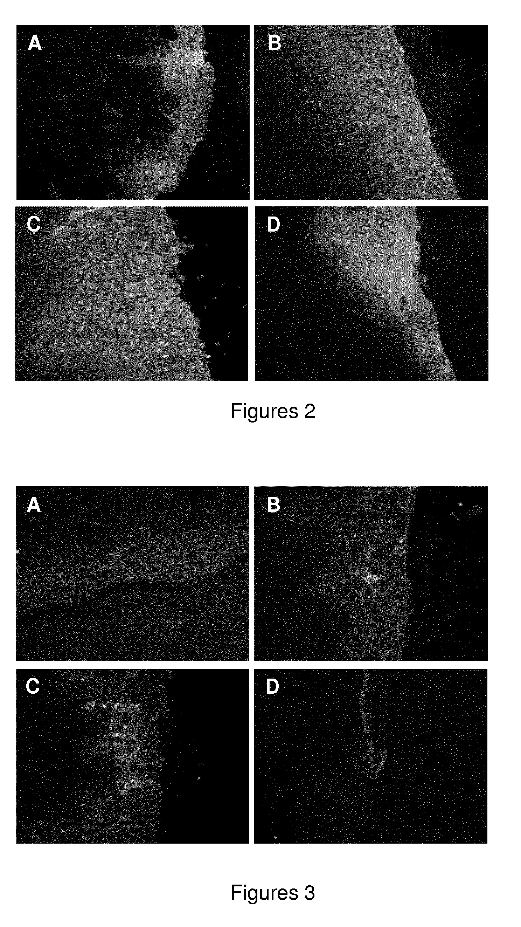

Figure 2A represents untreated SCC12B2-derived 3D reconstructed skin.
Figure 2B represents SCC12B2-derived 3D reconstructed skin treated by AZT (100 µM) for 21 days:
Figure 2C represents SCC12B2-derived 3D reconstructed skin treated by Lamivudine (100 µM) for 21 days.
Figure 2D represents SCC12B2-derived 3D reconstructed skin treated by AZT (100 µM) and Lamivudine (100 µM) for 21 days.
White staining denoted psoriasine expression.
**Figures 3A-D** represent the expression of cytokeratin 10 on SCC12B2-derived 3D reconstructed skin detected by immunofluorescence.
Figure 3A. represents untreated SCC12B2-derived 3D reconstructed skin.
Figure 3B represents SCC12B2-derived 3D reconstructed skin treated by AZT (100 µM) for 21 days:
Figure 3C represents SCC12B2-derived 3D reconstructed skin treated by lamividune (100 µM) for 21 days;
Figure 3D represents SCC12B2-derived 3D reconstructed skin treated with a combination of AZT (100 µM) and lamivudine (100 µM).
   White staining denoted cytokeratin 10 expression.

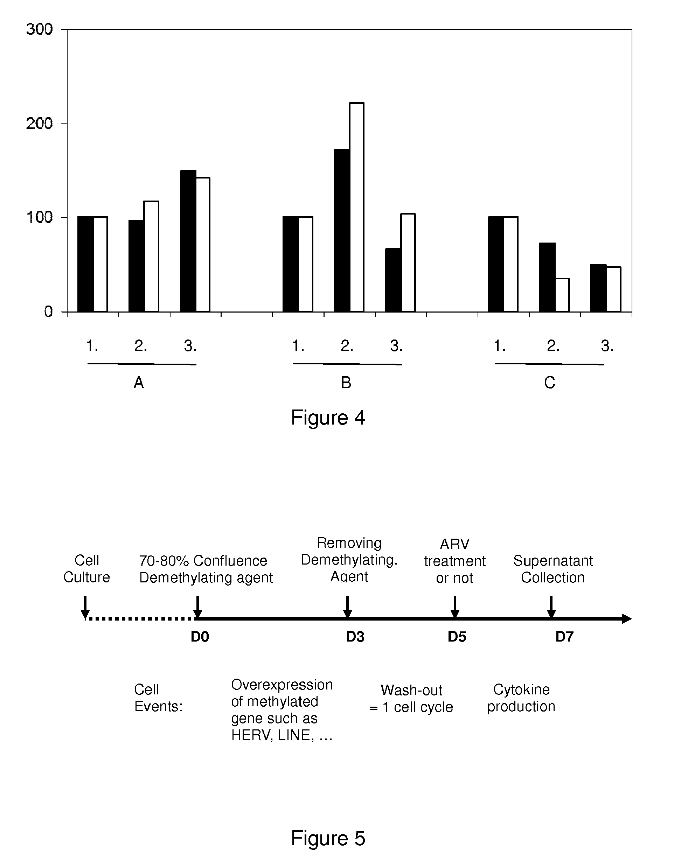

**Figure 4** represents a histogram showing the level of endogenous RT activity in 3D reconstructed skin from various cell lines: PC F2 (A), PC B2 (B) or PC KN (C). PC F2 were obtained from the SCC12F2 cell line; PC B2 from the SCC12B2 cell line and PC KN from primary cultured human keratinocytes. Treatment were either azidothymidine (AZT; 2.) or efavirenz (3.) compared to the control (untreated; 1.).
**Figure 5** represents a fow chart of the different treatments that aim at mimicking the initiating events of the psoriatic lesions

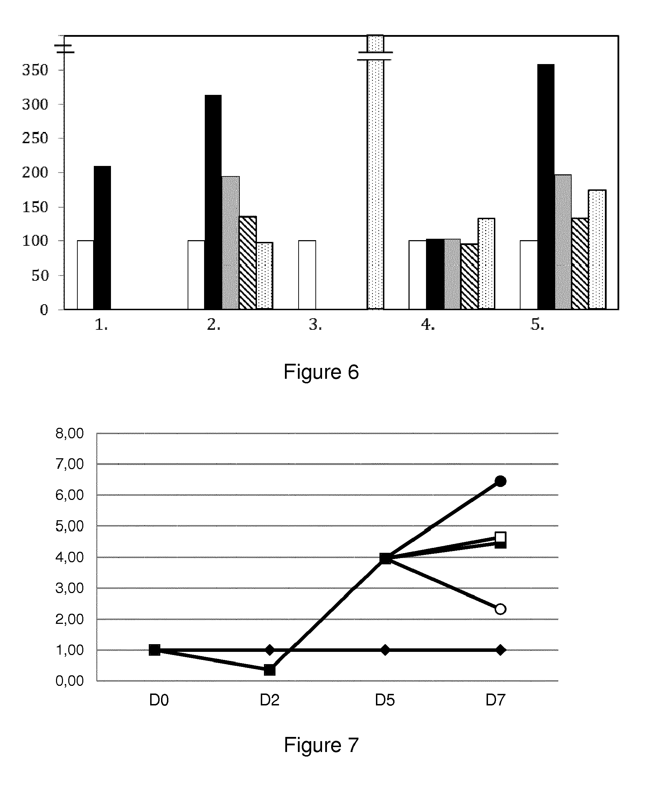

**Figure 6** represents a histogram showing the level of the indicated cytokine production by keratinocytes at Day 7 of the protocol described in figure 5. Data were normalized versus control condition (fold increase in arbitrary unit). 1: IL-17 A; 2: IFN-γ; 3: TNF-α, 4: IL-1 RA and 5: IP-10. White columns represent the expression level in control cells. Black columns represent the expression level in cells treated with deoxyuridine, Grey columns represent the expression level in cells treated with desoxyuridine and Eviplera®. Hatched columns represent the expression level in cells treated with desoxyuridine and rilpivirine. Columns with dots represent the expression level in cells treated with desoxyuridine and efavirenz.
**Figure 7** represents a graph showing time course (D0: Day 0; D2: Day 2; D5: Day 5; D7: Day 7) of the level of IL-6 production (fold increase) by keratinocytes according to the protocol described in figure 5. The expression is normalized to control at the corresponding time point. Diamond shape = untreated cells; Filled square = demethylating treated cells; Open square = treated with AZT; Filled circle = treated with lamivudine; Open circle = treated with Eviplera®.

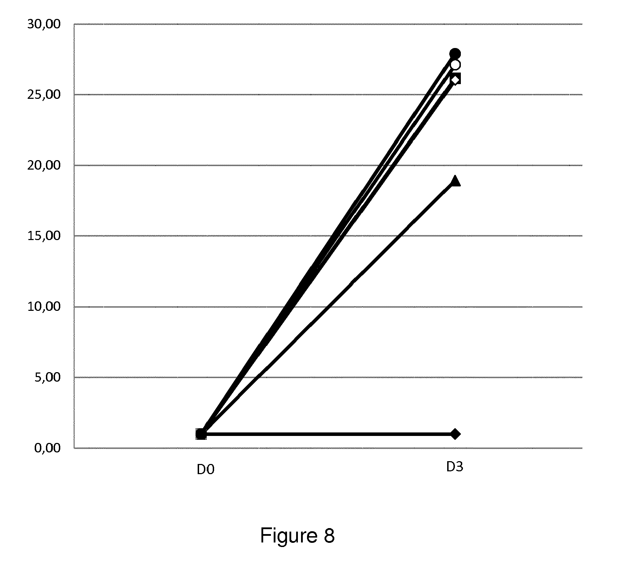

**Figure 8** represents a graph showing effects of ARVs upon simultaneous stimulation of RT activities on the production of IL-6 by keratinocytes. Values are normalized versus control. Filled diamond shape = control; Filled square = treated with demethylating agent; Triangle = treated with demethylating agent + Eviplera®; open diamond shape = treated with demethylating agent + lamivudine; Open circle = treated with demethylating agent + AZT; Filled circle = treated with demethylating agent + AZT + Lamivudine.

### EXAMPLES

### Introduction

Psoriasis is a T-lymphocyte mediated auto-inflammatory chronic skin disease, characterized by cutaneous erythemateous inflammatory lesions associating dermal infiltrate, epidermic hyperplasia and hyper and parakeratosis, evolving by periods of exacerbation and remission and affecting 1 to 3 % of the world population. Both lesion initiation and maintenance mechanisms remain to be identified. Over time therapeutic strategies of psoriasis have reflected its pathogenesis understanding, but with a poor benefits/risks balance overall.

The current knowledge of psoriasis pathophysiology highlights the prominent role of polarized Th17 lymphocytes. Secreted cytokines stimulate keratinocytes proliferation, secreting in return another cytokines set that stimulates lymphocyte activation. Once this cross-talk is initiated, lesions are generated. Targeting this auto-inflammatory maintenance loop, anti-cytokine biotherapies have shown encouraging results. But their incomplete efficiency in space and/or time in psoriasis, mainly due to frequent therapeutic escape, and their important adverse effects, questioning on their mid-term safety mandatory ask, new and more secure therapeutic options, targeting not only inflammation but factors triggering inflammation.

The question of psoriasis primum movens is still unanswered. Recently, increased amount of cytosolic DNA have been shown to promote cutaneous inflammation through TLR pathways activation. In the same way, the inventors found that endogenous reverse transcriptase activities were significantly higher in psoriasis lesional skin versus non lesional skin. These activities were issued of both the Mg²⁺ and the Mn²⁺ dependent reverse transcriptases.

Human epidermis, and more precisely the keratincoyte, is a unique model that includes stem cell populations and the various commitments towards the terminal differentiation stage. All these steps are advantageously recreated *in vitro* either in 2D culture model or in 3D culture model.

RT encoding genes have been detected in skin and increased expressions were found in hyperproliferative but benign skin lesion of patient affected by psoriasis. Interestingly, psoriatic epidermis is also characterised by an abnormal differentiation program, cells being not capable to generate a mature stratum corneum.

Moreover, the inventors showed that these RT were functional and a baseline activity was detectable in normal skin extracts. The previous increase expression was followed by an increase RT activity. These observations prompted the inventors to investigate the cellular functions of RT proteins by testing various classes of RT inhibitors on different primary cell culture conditions and keratinocyte cell lines.

The evaluation of RTI on psoriatic characteristics is based on three independent molecular and cellular models:
i) inhibition of the endogenous RT activity isolated from normal keratinocytes or keratinocyte cell lines,
ii) cell differentiation of normal keratinocytes or keratinocyte cell lines and
iii) regulation of cytokine production by normal keratinocytes or keratinocyte cell lines under RTI treatment.

These models represent discrete characteristics of the psoriatic lesion and allows to study, *in vitro* or *ex vivo* psoriasis mechanisms, since no animal models are available to date.

Cell hyperproliferation is a well-known characteristic of psoriasis. It is clearly accepted that keratinocytes divide 10 to 12 times more than in a normal epidermis which represent a turn-over even higher than in a cutaneous malignant tumors. At the dose tested below, the inventors demonstrated that ARVs were not cytotoxic and did not impaired cell proliferation. Therefore, effects described below should be regarded as direct effect on the mentioned cell process.

### Example 1 - Cell differentiation is impaired in the psoriatic lesion.

Effects of the composition according to the invention on cell differentiation was assessed via two cellular models:

### An organotypic culture model that recreates epidermis in 3 dimensions (3D).

### Material and Methods:

Briefly, various keratinocyte cell lines (SCC12B2, SCC12F2 [Weichselbaum, R. R., et al. Int. J. Radiation .Oncol. Biol. Phys., 14: 907-912, 1988] and primary cultures of keratinocytes were seeded onto a De-epidermized Dermis (DED) using a silicon ring (4-mm diameter).

DED were obtained from abdominoplasty and prepared as previously described *[*Mils V, et al.. Differentiation, 1994 Nov;58(1):77-86].). Keratinocytes were allowed to adhere to dermal equivalents for 2 h. The cultures were then submerged for 2 days in a culture medium consisting of Ham's F12/DMEM (1/3; vol./vol.) supplemented with 10% fetal calf serum (BioMedia, Boussens, France), 1% penicillin/streptomycin (Invitrogen, Cergy Pontoise, France), 0.4 µg.mL⁻¹ hydrocortisone, 5 µg.mL⁻¹ insulin, 10⁻¹⁰ M cholera toxin, and 10 ng.mL⁻¹ EGF (Sigma-Aldrich, Saint-Quentin-Fallavier, France). ARV were supplemented to the medium at the concentration of 10 and 100 µM. Treatments were applied both at the initiation of the culture and at day 11 and maintained until the end of the experiment. Finally, the cultures were placed on the stainless-steel grid at the air-liquid interface and cultured over a period of 21 days.

For detection of markers, whole 3D cultures were embedded in OCT (Miles, Zoeterwoude, The Netherlands) and snap-frozen in liquid nitrogen vapor. Sections of DEDs (6 µm) were obtained using a cryomicrotome (Leica, Wetzlar, Germany), fixed in acetone:methanol (1:1, vol./vol.) at 4°C for 20 min, rehydrated in PBS and incubated with a blocking solution (PBS containing 0.1% gelatin) for 2 h in a humidified chamber. Sections were then incubated overnight at 4°C with the primary antibodies. After several washes and incubation with an appropriate FITC conjugated secondary antibody for 1 h, sections were counterstained with Hoescht 33258 for nuclear detection. Negative controls consisted of omitting the primary antibody. Sections were then examined under a TE300 microscope fitted with a DMX1200 digital camera (Nikon, Tokyo, Japan).

Various markers have tested to characterize the reconstructed epidermis. This includes cytokeratin 1, 5, 6, 8, 10, 14, 16, 18 and 19, integrin α2, α3, α1, E-cadherin, involucrin, fillagrin.

The following ARV or combinations of ARV were tested: Zidovudine (AZT), Lamivudine, Efavirenz, Etravirine, Lopinavir + Ritonavir, Tenofovir, Rilpivirine, Emtricitabine +Tenofovir, Emtricitabine + Tenofovir + Rilpivirine.

### Results:

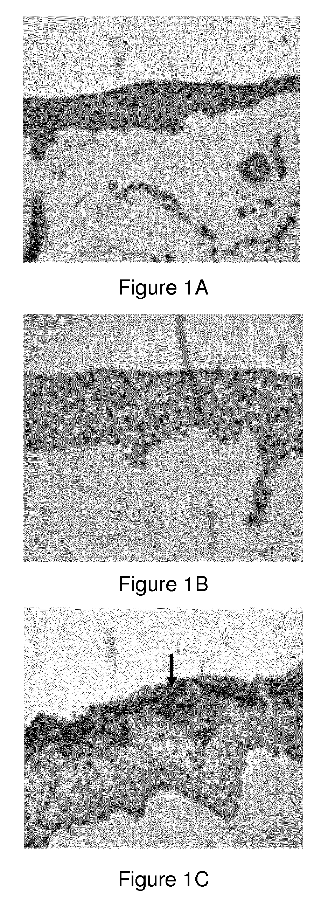

As shown in **figures 1A-C****,** AZT had no effect on cell differentiation whereas Efavirenz (NNRTI) reverses the "tumoral" phenotype of SCC12B2 cell line. Eosinophilic cells appear in the top layers and basal cells were organised in "palissade". The specific expression of cytokeratin 1 and 10 was noted in SCC12B2 skin culture. These keratins are restrictively expressed in differentiated cells i.e. in cells that withdraw from cell proliferation.

Based on this approach, others ARVs, or combinations of ARVs have been tested. Observed effects are listed in the following table:

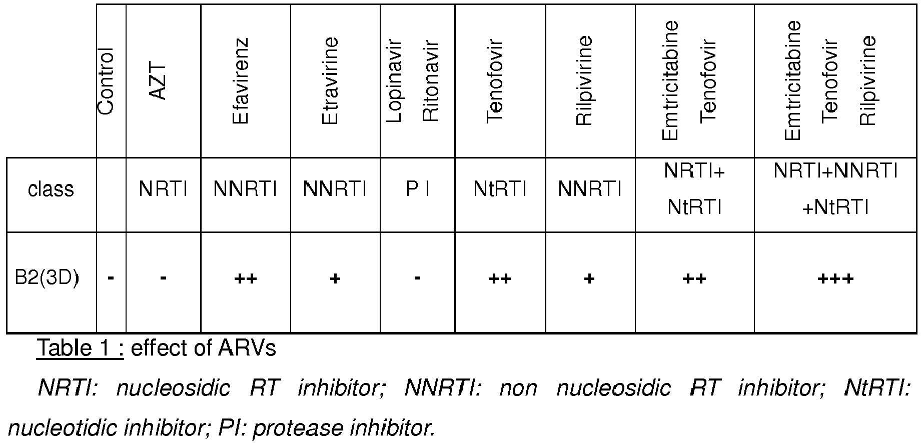

These results show that AZT, etravirine, lopinavir + ritonavir and rilpivirine have no or minor effects, whereas efavirenz, tenofovir, or combinations of said ARV impact cell differentiation.

To confim that AZT and lamivudine have no effects on cell differentiation, specific staining for psoriasine and cytokeratin 10 have been tested. The results are shown in figures 2 and 3.

Neither AZT, nor lamivudine has effect on cell differentiation.

These data confirm that only specific ARV, and combination thereof are efficient in psoriasis model. This applies also for chronic inflammatory diseases.

### Example 2 - Inhibition of the endogenous RT activity

Endogenous RT activities are increased in the psoriatic lesion when compared to non lesional psoriatic skin or normal skin extracts. They are both Mg⁺⁺ dependent and Mn⁺⁺ dependent RT activities which suggest that more than one type of RT are activated in the lesion. The origin and the nature of these proteins are unknown but considering that the disease is not transmissible horizontally they should be attributed to endogenous proteins. In fact several cellular proteins including telomerase, LINE ORF2 and RT from human endogenous retroviruses (HERV) harbour a reverse transcriptase activity. LINE ORF2 is a Mg²⁺-dependent RT, like RTs of the HERVs belonging to the MMTV-like group. The MLV-like HERV group harbours a Mn²⁺-dependent RT. Effects of ARV on these activities was assessed in 3D culture models: Various reconstructed skin were assessed for RT inhibition through RTI treatment. They were generated from SSC12F2 and SCC12B2 cell lines and from normal primary keratinocytes (KN).

Reverse transcriptase assay : Protein extracts were prepared by crushing the cryostat skin sections (100 µm) in liquid nitrogen, incubating in ice-cold lysis buffer (10 mM Tris-HCl pH 7.5, 1 mM MgCl₂, 1 mM EDTA, 0.1 mM PMSF, 5 mM β-mercaptoethanol, 0.5 % CHAPS, 10 % glycerol, 1 % NP40) and then subjected to three freeze/thaw cycles (LN₂/4°C). After 30 min centrifugation at 14,000 rpm at 4°C, supernatants were stored at -80°C until used. RT activities were quantified with colorimetric highly sensitive Mn²⁺ and Mg²⁺ RT kit (Cavidi, Uppsala, Sweden) according to the manufacturer's instructions. Briefly, 10 µl of extract were incubated overnight at 33°C with 140 µl of reaction buffer. After intensive washes, incorporated BrDU was revealed using alkaline phosphatase-conjugate antibody followed by alkaline phosphatase substrate reaction and optical reading at 405 nm (Spectra, SLT Labinstruments, Austria). Standardised MMLV or MMTV RT reaction was done in parallel in separated well. RT values of all extracts were then expressed in MMLV or MMTV RT units per µg of proteins.

Within the conditions tested, efavirenz decreased endogenous RT activities of both type i.e. Mn²⁺ and Mg²⁺ dependent, of 3 out of 4 reconstructed epidermis (Figure 4). Inhibition could represent 65% of the initial value. AZT, in the other hand, appeared less effective.

As an overall conclusion, ARVs previously designed to inhibit RT from infectious viruses, are also inhibitor for the endogenous RT.

### Example 3 - Inflammation

In order to recreate the series of events that lead to the generation of the psoriatic lesion, the inventors set up the experimental model depicted in figure 5. In the first part, the inventors aim at generating RT activities and in the second part at evaluating the consequence in term of cytokine production.

Results are shown in **figure 6****.**

Amongst the tested cytokine, IL17A, IFN-α and IP10 production were increased after the demethylation process. TNF-α and IL-1RA did not increased in this condition. After such treatment, Eviplera^{®}, rilpivirine and efavirenz were efficient at reducing the level of production of the former set of cytokine. IL17A expression was undetectable when the two other cytokine expression were decreased by more than 50%. Of note, efavirenz treatment induced a potent induction of TNF-α production.

Used ARVs (i.e ARV according to the invention) can restore a basal level of cytokine expression in keratinocytes after treatment with demethylating agent. Eviplera^{®} or rilpivirine may prevent skin inflammation.

In order to confirm the effects of the ARV treatment, secretion of IL-6 was assessed upon RT induction during the time course of the experiments (figure 5). Results are shown in **figure 7****.**

These results show that: (i) IL-6 is induced after demethylating treatment, (ii) neither AZT nor lamivudine have effect on the synthesis of IL-6 upon this condition and (iii) Eviplera^{®} reduces the synthesis of IL-6 by 66%.

In another set of experiments, cells received concomitantly a treatment that stimulated RT activity and a dose of different classes of ARVs. Again, IL-6 production was assessed and results are shown in **figure 8****.**

AZT, lamivudine or a combination of said RTI have no effect on the synthesis of IL-6 whereas Eviplera^{®} reduces the synthesis of IL-6 by 30% under this condition.

### Example 4 - Other pathologies or models

The chronic auto-inflammatory human diseases that are eligible for such concept fulfill two criteria: (i) have an increase endogenous RT activity and (ii) have an increase cytosolic DNA content.

A series of various human diseases as listed above, including polyarthritis rhumatoid, sclerodermia, were assessed for endogenous RT activity as described in example 2.

Cytosolic DNA content was performed by Taqman-based quantitative real-time PCR according to Coufal et al. (2009) with minor modifications (100 or 500 pg DNA as starting material and single amplicon analysis. Primers are listed below. Quantification was performed in triplicate. A non-parametric Mann-Whitney U test was employed for two group comparison and p < 0.05 was considered significant.

### Human L1 ORF2

PCR primer_F TGCGGAGAAATAGGAACACTTTT (SEQ ID NO: 1)
PCR primer_R TGAGGAATCGCCACACTGACT (SEQID NO: 2)
Taqman probe CTGTAAACTAGTTCAACCATT (SEQID NO: 3)

### Human L1 ORF1

PCR primer_F CAAACACCGCATATTCTCACTCA (SEQID NO: 4)
PCR primer_R CTTCCTGTGTCCATGTGATCTCA (SEQID NO: 5)
Taqman probe AGGTGGGAATTGAAC (SEQID NO: 6)

### HSATA

PCR primer_F GGTCAATGGCAGAAAAGGAAAT (SEQID NO: 7)
PCR primer_R CGCAGTTTGTGGGAATGATTC (SEQID NO: 8)
Taqman probe TCTTCGTTTCAAAACTAG (SEQID NO: 9)

### HHERVH

PCR primer_F AATGGCCCCACCCCTATCT (SEQID NO: 10)
PCR primer_R GCGGGCTGAGTCCGAAA (SEQID NO: 11)
Taqman probe CCCTTCGCTGACTCTC (SEQID NO: 12)

A second approach consists to use animal models to assess the efficacy of an ARV, in particular RTI treatment.

For psoriasis, imiquimod-treated BALB-C mice are used.

Systemic lupus erythematosus can be reproduced by treating mice with hydralazine or isoniazid for 6 months. Systemic Sclerosis model is derived from BALB-C mice treated with daily injection of hydroxyl radicals, hypochlorous acid, peroxynitrites or superoxide anions for 6 weeks.

These animals received an ARV, in particular, a RTI, treatment either during the induction of the lesions or once the lesions are acquired. Evaluation of the efficacy of the treatment is studied according to the characteristics of the said disease.

## Claims

1. Composition comprising :
- an effective amount of a first antiretroviral agent, said first antiretroviral agent being tenofovir, or a salt or a solvate or a prodrug thereof, and
- an effective amount of at least a second antiretroviral agent, said second antiretroviral agent been chosen among the group consisting of: lamivudine, rilpivirine, efavirenz, raltegravir, or a combination thereof, or a salt or a solvate or a prodrug of the above,
for its use for the prevention or the treatment of chronic inflammatory diseases.

2. Composition for its use according to claim 1, wherein said auto-inflammatory diseases is chosen among the group consisting of: psoriasis, sclerodermia, schizophrenia, autism, Alzheimer's disease, central nervous system inflammation, Parkinson's Disease, dementia (various forms), multiple sclerosis, Sjrögren syndrome, obesity, Hashimoto's disease, diabetes (Type 1 and Type 2), atherosclerosis, neonatal onset multisystem inflammatory disease, (also known as NOMID), Chronic Neurologic Cutaneous and Articular Syndrome (or CINCA), rheumatoid arthritis, juvenile idiopathic arthritis, osteoarthritis, systemic lupus erythematosus, cutaneous lupus erythematosus, organ inflammation (eg. myocarditis, asthma, nephritis, colitis), inflammatory bowel disease (IBD), inflammatory bowel syndrome (IBS), Crohn's disease, primary biliary cirrhosis, Chronic Ulcerative Colitis, celiac disease, inflammatory lung disease, chronic obstructive pulmonary disease, idiopathic emphysema and idiopathic pulmonary fibrosis, Aicardi-Goutières syndrome, idiopathic autoimmune chronic active hepatitis (IACAH).

3. Composition for its use according to claim 1 or 2, wherein said first antiretroviral agent and said second medical agent are combined for a simultaneous, separate or sequential use.

4. Composition for its use according to anyone of claims 1 to 3, comprising :
- an effective amount of a first antiretroviral agent, said first antiretroviral agent being tenofovir or rilpivirine, or a salt or a solvate or a prodrug thereof,
- an effective amount of a second antiretroviral agent, said second antiretroviral agent, been chosen among the group consisting of: lamivudine, rilpivirine, efavirenz, integrase inhibitors, or a combination thereof, or a salt or a solvate or a prodrug of the above, and possibly
- an effective amount of a third antiretroviral agent, said third antiretroviral agent been chosen among the group consisting of: lamivudine, rilpivirine, efavirenz, integrase inhibitors, or a combination thereof, or a salt or a solvate or a prodrug of the above,
said second and third antiretroviral agent being different.

5. Composition for its use according to anyone of claims 1 to 4, comprising :
- an effective amount of a first antiretroviral agent, said first antiretroviral agent being tenofovir, or a salt or a solvate or a prodrug thereof,
- an effective amount of a second antiretroviral agent, said second antiretroviral agent being lamivudine, or a salt or a solvate or a prodrug thereof, and possibly
- an effective amount of a third antiretroviral agent, said third antiretroviral agent being rilpivirine, or a salt or a solvate or a prodrug thereof.

6. Composition for its use according to anyone of claims 1 to 5, wherein the effective amount of the first antiretroviral agent is from 1 µM to 10 mM.

7. Composition for its use according to anyone of claims 1 to 6, wherein the effective amount of the second antiretroviral agent is from 1 µM to 100 µM.

8. Composition for its use according to anyone of claims 1 to 7, comprising
- an effective amount of a first antiretroviral agent, said first antiretroviral agent being tenofovir, or a salt or a solvate or a prodrug thereof,
- an effective amount of a second antiretroviral agent, said second antiretroviral agent being lamivudine, or a salt or a solvate or a prodrug thereof, and
- an effective amount of a third antiretroviral agent, said third antiretroviral agent being rilpivirine, or a salt or a solvate or a prodrug thereof,
wherein said auto-inflammatory disease is psoriasis.

9. Composition for its use according to anyone of claims 1 to 8, in association with a vitamin D derivative, or a retinoid compound, or corticoid, tar and anthracene derivative, methotrexate, ciclosporin, fumaric acid derivatives, immunosuppressive treatment (such as tacrolimus or sirolimus) and any anti cytokine/cytokine receptor antibody, and wherein said pathology is psoriasis.


SEQUENCE LISTING

<110>  Université Montpellier 1
       Institut National de la Santé et de la Recherche Médicale

<120>  USE OF ANTIRETROVIRAL AGENTS FOR TREATING PATHOLOGIES

<130>  BR51052

<160>  12

<170>  PatentIn version 3.5

<210>  1
<211>  23
<212>  DNA
<213>  Artificial Sequence

<220>
<223>  primer forward for Human L1 ORF2

<400>  1
tgcggagaaa taggaacact ttt                                              23


<210>  2
<211>  21
<212>  DNA
<213>  Artificial Sequence

<220>
<223>  primer reverse for Human L1 ORF2

<400>  2
tgaggaatcg ccacactgac t                                                21


<210>  3
<211>  21
<212>  DNA
<213>  Artificial Sequence

<220>
<223>  Taqman probe for Human L1 ORF2

<400>  3
ctgtaaacta gttcaaccat t                                                21


<210>  4
<211>  23
<212>  DNA
<213>  Artificial Sequence

<220>
<223>  primer forward for Human L1 ORF1

<400>  4
caaacaccgc atattctcac tca                                              23


<210>  5
<211>  23

<212>  DNA
<213>  Artificial Sequence

<220>
<223>  primer reverse for Human L1 ORF1

<400>  5
cttcctgtgt ccatgtgatc tca                                              23


<210>  6
<211>  15
<212>  DNA
<213>  Artificial Sequence

<220>
<223>  Taqman probe for Human L1 ORF1

<400>  6
aggtgggaat tgaac                                                       15


<210>  7
<211>  22
<212>  DNA
<213>  Artificial Sequence

<220>
<223>  primer forward for HSATA

<400>  7
ggtcaatggc agaaaaggaa at                                               22


<210>  8
<211>  21
<212>  DNA
<213>  Artificial Sequence

<220>
<223>  primer reverse for HSATA

<400>  8
cgcagtttgt gggaatgatt c                                                21


<210>  9
<211>  18
<212>  DNA
<213>  Artificial Sequence

<220>
<223>  Taqman probe for HSATA

<400>  9
tcttcgtttc aaaactag                                                    18


<210>  10
<211>  19
<212>  DNA
<213>  Artificial Sequence

<220>
<223>  primer forward for hHERV-H

<400>  10
   aatggcccca cccctatct                                                    19


<210>  11
<211>  17
<212>  DNA
<213>  Artificial Sequence

<220>
<223>  reverse primer for hHERV-H

<400>  11
gcgggctgag tccgaaa                                                       17


<210>  12
<211>  16
<212>  DNA
<213>  Artificial Sequence

<220>
<223>  Taqman probe for hHERV-H

<400>  12
cccttcgctg actctc                                                        16